# EUROPÄISCHE PATENTSCHRIFT

(11) **EP 2 445 880 B1**
(45) Veröffentlichungstag und Bekanntmachung des Hinweises auf die Patenterteilung: **23.01.2013**
(21) Anmeldenummer: 10747156.7
(22) Anmeldetag: 23.06.2010
(51) Int. Cl.: C07D 213/10

(54) **SYNTHESEPROZESS FÜR 3-METHYLPYRIDIN**
Synthesis process for 3-methylpyridine
Processus de synthèse pour 3 méthylpyridine

(30) Priorität: 24.06.2009 EP 09008251
(43) Veröffentlichungstag der Anmeldung: 02.05.2012
(73) Patentinhaber: Lonza Ltd, 3930 Visp (CH)
(72) Erfinder: ROEDERER, Detlef, CH-3900 Brig (CH); ZOLLINGER, Daniel, CH-3960 Sierre (CH); DE RIEDMATTEN, Jean-Yves, CH-1958 Uvrier (CH)
(86) Internationale Anmeldenummer: PCT/EP2010/003773
(87) Internationale Veröffentlichungsnummer: WO 2010/149352

(56) Entgegenhaltungen:
- FR-A- 2 161 128
- GB-A- 1 005 984
- US-A- 2 700 042
- J. I. GRAYSON, R. DINKEL: "244. An Improved Liquid-Phase Synthesis of Simple Alkylpyridines", HELVETICA CHIMICA ACTA, Bd. 67, Nr. 8, 1984, Seiten 2100-2110, XP002548839, in der Anmeldung erwähnt
- M. I. FARBEROV ET AL.: "Synthesis of pyridine compounds from paraldehyde and ammonia", ZHURNAL PRIKLADNOI KHIMII, Bd. 37, Nr. 3, 1964, Seiten 661-668, XP009123706,

## Beschreibung

Die vorliegende Erfindung betrifft ein Verfahren zur Herstellung von 3-Methylpyridin (3-Picolin) aus Formaldehyd, Paracetaldehyd, Ammoniak und Essigsäure.

3-Picolin ist eine farblose, brennbare Flüssigkeit, welche Verwendung als Lösungsmittel, bei der Herstellung von Pharmazeutika und Insektiziden sowie zur Synthese von Nikotinsäure und Nikotinamid findet.

Es sind verschiedene Synthesewege zur Herstellung von 3-Picolin bekannt, welche generell auf einer Additions-/Cyclisierungsreaktion von Aldehyd/Keton Mischungen mit einer Ammoniumverbindung beruhen. Diese Reaktionen können in der Gasphase oder in der flüssigen Phase sowie unter Verwendung eines Katalysators ablaufen.

Das erfindungsgemäße Verfahren baut auf der Veröffentlichung von Grayson, J. und Dinkel, R., "An improved Liquid-Phase Synthesis of Simple Alkylpyridines", Helvetica Chimica Acta, Vol. 67 (1984), S. 2100-2110, auf.

Die Autoren dieser Veröffentlichung beschreiben in Tabelle 2, S. 2108 u.a. die Synthese von 3-Picolin aus Acetaldehyd und Formaldehyd, wobei verschiedene Ammonium-Quellen hinsichtlich des Gehalts an 3-Picolin sowie diverser, unerwünschter Nebenprodukte verglichen werden.

Insbesondere wird gezeigt, dass bei der Verwendung von Ammoniumacetat eine Ausbeute von 44 % erreicht wird, wobei als Hauptnebenprodukt 3-Ethylpyridin in einer Menge von 18 % entsteht.

Es ist die Aufgabe der vorliegenden Erfindung, das Verfahren von Grayson und Dinkel in Bezug auf die Ausbeute an 3-Picolin, Reduktion von 3-Ethylpyridin als Hauptnebenprodukt sowie Raum/Zeit-Ausbeute zu verbessern.

Dieses Problem wird durch das erfindungsgemäße Verfahren zur Synthese von 3-Methylpyridin aus Formaldehyd, Paracetaldehyd, Ammoniak und Essigsäure gelöst, dadurch gekennzeichnet, dass die genannten Verbindungen zur Reaktion gebracht werden und das Verfahren die folgenden Parameter umfasst:
a) eine Reaktionstemperatur von 260-300°C;
b) ein molares Verhältnis von Formaldehyd und Paracetaldehyd von 0.7-1.4 Mol/Mol;
c) eine Ammoniak-Konzentration von 10-20 Gew.-%;
d) eine Essigsäure-Konzentration von 4-20 Gew.-%;
e) eine Paracetaldehyd-Konzentration von 0.4-1.6 Mol/kg;
f) eine Verweilzeit von 10-30 Minuten im Falle einer kontinuierlichen Reaktionsführung sowie 10 bis 90 Minuten im Falle einer diskontinuierlichen Reaktionsführung; sowie
g) einen Reaktionsdruck von 30 bis 130 bar

Es kann erfindungsgemäß bevorzugt sein, dass die Reaktion in einem Reaktor stattfindet. Besonders bevorzugt sind hierbei Systeme mit hoher Durchmischungseffizienz wie Rührwerke sowie kontinuierliche Durchflussrührkessel und diskontinuierliche Rührkessel, ganz besonders bevorzugt sind Loop-Reaktoren und Jet-Loop-Reaktoren.

Loop- und Jet-Loop-Reaktoren sind erfindungsgemäß dadurch gekennzeichnet, dass die entsprechenden Reaktanden mit Katalyt-Lösung unter kontinuierlicher Fahrweise zur Reaktion gebracht werden. Ein wesentlicher Vorteil von Jet Loop Reaktoren bei der Herstellung von 3-Picolin ist die intensivere und schnellere Flüssigkeitsvermischung bei hoher Umlaufströmung und somit ein erhöhter Wärme- und Stoffübergang als in einem Rührkessel. Bevorzugt wird die erfindungsgemäße Reaktion in einem Strahlzonen-Schlaufenreaktor durchgeführt. Ein weiterer Vorteil ergibt sich bei strahlgetriebenen [Düsen] Schlaufenreaktoren durch eine feinere Dispergierung der zugespeisten Phasen und somit einer größeren spezifischen Phasengrenzfläche.

Des Weiteren kann es erfindungsgemäß bevorzugt sein, dass im Rahmen der Rezirkulierung des Katalysators bei der Reaktion entstehende Nebenprodukte entfernt werden. Hierfür kommen grundsätzlich die im Stand der Technik bekannten Möglichkeiten in Betracht, wie z.B. Extraktion und Rektifikation. Hierbei ist die Destillation erfindungsgemäß besonders bevorzugt.

Erfindungsgemäß können Ammoniak und Formaldehyd sowohl in molekularer Form als auch in Form ihres Additionsproduktes Hexamethylentetramin (Urotropin) zugegeben werden.

Das erfindungsgemäße Verfahren hebt sich vom Stand der Technik gemäß Dinkel et al. u.a. dadurch ab, dass es eine höhere Selektivität im Hinblick auf die Bildung von 3-Methylpyridin mit einer höheren Raum-/Zeit-Ausbeute kombiniert. Damit wurde ein besonderer technischer Fortschritt erzielt, da normalerweise bei der vorliegenden Reaktion eine Erhöhung der Raum-/Zeit-Ausbeute mit einer geringeren Selektivität einhergeht.

Das erfindungsgemäße Verfahren wird durch das folgende Beispiel näher erläutert.

### Beispiel 1

Die Reaktion findet kontinuierlich in einem sehr gut durchgemischten 100 Liter Reaktor statt. Die Zugabe der Katalytlösung (Gemisch Wasser, Ammoniak und Essigsäure) sowie der Edukte (Paracetaldehyd und Formalin) erfolgt mittels Pumpen.

261 kg/h der Katalytlösung (75 Gew.-% Wasser, 15 Gew.-% Ammoniak und 10 Gew.-% Essigsäure) werden mittels Hochdruckpumpe in den Reaktor befördert. Gleichzeitig werden ebenfalls kontinuierlich über Hochdruckpumpen 13 kg/h Paracetaldehyd und 26.8 kg/h Formalinlösung [37.4 Gew.-%] zudosiert. Die Temperatur im Reaktor wird auf 278°C und der Reaktordruck auf 100 bar gehalten. Bei einer Verweilzeit im Reaktor von 20 Minuten erhält man am Reaktoraustritt eine Rohlösung, die 10.02 kg/h 3-Picolin enthält bei einer gleichzeitigen Produktion von 0.37 kg/h 3-Ethylpyridin. Unter diesen Bedingungen wird eine 3-Picolin Ausbeute von 64.6% (basierend auf Formaldehyd) und eine 3-Ethylpyridin Ausbeute von 3.5% (basierend auf Acetaldehyd) erzielt. Alle Pyridinbasen werden per GC gemessen.

## Patentansprüche

1. Verfahren zur Synthese von 3-Methylpyridin aus Formaldehyd, Paracetaldehyd, Ammoniak und Essigsäure, **dadurch gekennzeichnet, dass** die genannten Verbindungen zur Reaktion gebracht werden und das Verfahren die folgenden Parameter umfasst:
a) eine Reaktionstemperatur von 260-300°C;
b) ein molares Verhältnis von Formaldehyd und Paracetaldehyd von 0.7-1.4 Mol/Mol;
c) eine Ammoniak-Konzentration von 10-20 Gew.-%;
d) eine Essigsäure-Konzentration von 4-20 Gew.-%;
e) eine Paracetaldehyd-Konzentration von 0.4-1.6 Mol/kg;
f) eine Verweilzeit von 10-30 Minuten bei einer kontinuierlichen Reaktionsführung sowie 10 bis 90 Minuten im Falle einer diskontinuierlichen Reaktionsführung; sowie
g) einen Reaktionsdruck von 30 bis 130 bar

2. Verfahren gemäß Anspruch 1, **dadurch gekennzeichnet, dass** die Reaktion in einem Reaktor mit hoher Durchmischungseffizienz stattfindet.

3. Verfahren gemäß Anspruch 2, **dadurch gekennzeichnet, dass** es sich um einen kontinuierlichen oder diskontinuierlichen Durchflussrührkessel handelt.

4. Verfahren gemäß Anspruch 2, **dadurch gekennzeichnet, dass** es sich um einen Loop-Reaktor handelt.

5. Verfahren gemäß mindestens einem der Ansprüche 1-4, **dadurch gekennzeichnet, dass** im Rahmen einer Rezirkulierung des Katalysators unerwünschte Nebenprodukte entfernt werden.

6. Verfahren gemäß Anspruch 5, **dadurch gekennzeichnet, dass** besagte Nebenprodukte mittels Rektifikation und Extraktion entfernt werden.

7. Verfahren gemäß mindestens einem der Ansprüche 1-6, wobei die Raum-/ZeitAusbeute an 3-Methylpyridin mehr als 50 kg/m³*h, bevorzugt mehr als 80 kg/m³*h und besonders bevorzugt mindestens 100 kg/m³*h beträgt.

8. Verfahren gemäß mindestens einem der Ansprüche 1-7, wobei die Ausbeute an 3-Methylpyridin mindestens 64% (bezogen auf Formaldehyd) und die Ausbeute an Ethylpyridin höchstens 4% (bezogen auf Paracetaldehyd) beträgt.

## Claims

1. A process for the synthesis of 3-methylpyridine from formaldehyde, paraldehyde, ammonia and acetic acid, **characterized in that** said compounds are reacted and said process comprises the following parameters:
a) a reaction temperature of 260-300 °C;
b) a molar ratio of formaldehyde and paraldehyde of 0.7-1.4 mol/mol;
c) an ammonia concentration of 10-20 weight-%;
d) an acetic acid concentration of 4-20 weight-%;
e) a paraldehyde concentration of 0.4-1.6 mol/kg;
f) a retention time of 10-30 minutes in case of a continuous reaction and 10-90 minutes in case of a discontinuous reaction; and
g) a reaction pressure of 30-130 bar.

2. The process of claim 1, **characterized in that** the reaction takes place in a reactor having a high efficiency of mixing.

3. The process of claim 2, **characterized in that** said reactor is a continuous or discontinuous flow-through stirred vessel.

4. The process of claim 2, **characterized in that** said reactor is a loop reactor.

5. The process of at least one of claims 1-4, **characterized in that** unwanted byproducts are removed in the process of recycling the catalyst.

6. The process of claim 5, **characterized in that** said side products are removed by rectification or extraction.

7. The process of at least one of claims 1-6, whereby the space/time yield of 3-methylpyridine is more than 50 kg/m³·h, preferably more than 80 kg/m³·h and most preferably more than 100 kg/m³·h.

8. The process of at least one of claims 1-7, whereby the 3-methylpyridine yield is at least 64% (based on formaldehyde) and the 3-ethylpyridine yield is at most 4% (based on paraldehyde).

## Revendications

1. Procédé de synthèse de 3-méthylpyridine à partir de formaldéhyde, de paraldéhyde, d'ammoniac et d'acide acétique, **caractérisé en ce que** les composés mentionnés sont mis en réaction et le procédé comprend les paramètres suivants :
a) une température de réaction de 260 à 300 °C ;
b) un rapport molaire entre le formaldéhyde et le paraldéhyde de 0,7 à 1,4 mol/mol ;
c) une concentration en ammoniac de 10 à 20 % en poids ;
d) une concentration en acide acétique de 4 à 20 % en poids ;
e) une concentration en paraldéhyde de 0,4 à 1,6 mol/kg ;
f) un temps de séjour de 10 à 30 minutes lors d'une réalisation continue de la réaction, et de 10 à 90 minutes en cas d'une réalisation discontinue de la réaction ; et
g) une pression de réaction de 30 à 130 bar.

2. Procédé selon la revendication 1, **caractérisé en ce que** la réaction a lieu dans un réacteur à efficacité de mélange élevée.

3. Procédé selon la revendication 2, **caractérisé en ce qu'**il s'agit d'une cuve agitée à écoulement continue ou discontinue.

4. Procédé selon la revendication 2, **caractérisé en ce qu'**il s'agit d'un réacteur à boucle.

5. Procédé selon au moins l'une quelconque des revendications 1 à 4, **caractérisé en ce que** des produits secondaires indésirables sont éliminés dans le cadre d'un recyclage du catalyseur.

6. Procédé selon la revendication 5, **caractérisé en ce que** lesdits produits secondaires sont éliminés par rectification et extraction.

7. Procédé selon au moins l'une quelconque des revendications 1 à 6, **caractérisé en ce que** le rendement espace-temps en 3-méthylpyridine est supérieur à 50 kg/m³·h, de préférence supérieur à 80 kg/m³·h et de manière particulièrement préférée d'au moins 100 kg/m³·h.

8. Procédé selon au moins l'une quelconque des revendications 1 à 7, **caractérisé en ce que** le rendement en 3-méthylpyridine est d'au moins 64 % (par rapport au formaldéhyde) et le rendement en éthylpyridine est d'au plus 4 % (par rapport au paraldéhyde).
